(19) Europäisches Patentamt European Patent Office Office européen des brevets

(11) **EP 3 174 844 B1**

(12) **FASCICULE DE BREVET EUROPEEN**

(45) Date de publication et mention de la délivrance du brevet:
**07.11.2018 Bulletin 2018/45**

(21) Numéro de dépôt: **15735994.4**

(22) Date de dépôt: **22.06.2015**

(51) Int Cl.:
*C07C 67/08* *(2006.01)*      *C07C 69/54* *(2006.01)*
*C07C 67/54* *(2006.01)*

(86) Numéro de dépôt international:
**PCT/FR2015/051648**

(87) Numéro de publication internationale:
**WO 2016/016528 (04.02.2016 Gazette 2016/05)**

(54) **PROCÉDÉ PERFECTIONNE DE FABRICATION DE (MÉTH)ACRYLATES D'ALKYLE**

VERBESSERTES VERFAHREN ZUR HERSTELLUNG VON ALKYL(METH)ACRYLATEN

IMPROVED METHOD FOR PRODUCING ALKYL (METH)ACRYLATES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.07.2014 FR 1457262**

(43) Date de publication de la demande:
**07.06.2017 Bulletin 2017/23**

(73) Titulaire: **Arkema France**
**92700 Colombes (FR)**

(72) Inventeurs:
• **MORELIERE, Anne**
**F-57740 Longeville-les-St-Avold (FR)**
• **FRIMOUR, Gilles**
**F-57320 Dalstein (FR)**
• **PAUL, Jean-Michel**
**F-57070 Metz (FR)**
• **DENIS, Stephane**
**F-57660 Leyviller (FR)**

(74) Mandataire: **Bonnel, Claudine et al**
**ARKEMA France**
**Département Propriété Industrielle**
**420, rue d'Estienne d'Orves**
**92705 Colombes Cedex (FR)**

(56) Documents cités:
**EP-A1- 1 219 587     FR-A1- 2 739 850**
**US-A- 4 748 268**

**Description**

DOMAINE TECHNIQUE

La présente invention concerne la fabrication de (méth)acrylates d'alkyle par estérification directe de l'acide (méth)acrylique par l'alcool correspondant, cette réaction étant catalysée par une résine cationique.

Elle a plus particulièrement pour objet un procédé perfectionné de fabrication de (méth)acrylates d'alkyle en $C_4$-$C_{10}$, en particulier d'acrylate de 2-éthylhexyle, mettant en oeuvre de nouvelles conditions de réaction conduisant à une simplification du procédé et à une productivité élevée d'un produit répondant aux normes en matière de pureté.

ART ANTERIEUR ET PROBLEME TECHNIQUE

Les problèmes qui se posent lors de la fabrication des (méth)acrylates d'alkyle en $C_4$-$C_{10}$ par estérification directe de l'acide (méth)acrylique en présence d'une résine cationique comme catalyseur, sont le plus souvent liés à la complexité des étapes de purification nécessaires après l'étape réactionnelle pour obtenir un produit de haute pureté, au détriment en général de la productivité du procédé.

Afin de simplifier les opérations de purification en aval, il a été proposé dans le procédé de fabrication d'ester d'acide carboxylique insaturé décrit dans le document EP 1219587 au nom de la Demanderesse, de conduire la réaction d'estérification en faisant passer de manière ascendante le mélange des réactifs à travers un lit d'une résine cationique, dans une boucle de recirculation qui est associée à une cuve agitée dans laquelle est assuré le mélange des réactifs et à partir de laquelle est assurée l'élimination de l'eau de réaction sous la forme d'un azéotrope avec l'alcool estérifiant. La réaction d'estérification est effectuée en présence d'un léger excès d'alcool, en particulier avec un rapport molaire global acide/alcool compris entre 0,6 et 1. Selon un mode de réalisation, une réaction partielle en amont de la cuve agitée est effectuée en adressant le mélange des réactifs de manière ascendante à travers un second lit de la résine cationique comme catalyseur.

Bien que ce procédé conduise à des taux de conversion élevés et des sélectivités également élevées, il est apparu à la Demanderesse que la complexité de la zone réactionnelle et de son fonctionnement rendait le procédé difficile à mettre en oeuvre et ne permettait pas d'obtenir la pureté souhaitée pour le produit fini.

Dans le procédé de production d'esters méthacryliques par estérification directe catalysée par l'acide sulfurique, décrit dans le document US 4,748,268, le mélange réactionnel en sortie du réacteur est fractionné en continu dans une colonne de distillation, en un distillat de tête riche en ester produit, et en un flux de pied contenant les réactifs non réagis, de l'eau, des sous-produits non volatiles, le catalyseur et un peu d'ester. Ce flux de pied est en partie renvoyé dans le réacteur constituant ainsi une boucle de recirculation dans l'installation.

Après de nombreuses recherches, la Demanderesse a trouvé qu'il était possible de produire des esters (méth)acryliques de haute pureté par estérification directe dans une zone réactionnelle simplifiée, en utilisant un excès d'alcool pour la réaction d'estérification et en faisant recirculer une proportion importante du mélange réactionnel dans une boucle de recirculation ne comprenant que le réacteur d'estérification et une colonne de distillation qui élimine l'eau produite sous forme d'un azéotrope avec l'alcool estérifiant. De façon surprenante, ces conditions opératoires permettent d'optimiser le rendement de la réaction et d'éliminer efficacement l'eau produite par la réaction, minimisant ainsi les réactions secondaires responsables de la formation d'impuretés et de sous-produits lourds, et donc d'une baisse de la productivité du procédé.

La présente invention a donc pour objet un procédé perfectionné de fabrication de (méth)acrylates d'alkyle, simple à mettre en oeuvre, avec un rendement élevé et une faible génération de sous-produits lourds, ce qui simplifie les opérations de purification en aval. Par ailleurs, le produit purifié contient seulement de faibles traces d'impuretés liées à l'acide et à l'alcool mis en oeuvre.

RESUME DE L'INVENTION

La présente invention a pour objet un procédé de fabrication d'un (méth)acrylate d'alkyle en $C_4$-$C_{10}$, par esté-rification directe d'acide (méth)acrylique par l'alcool correspondant, l'eau de réaction étant éliminée sous la forme d'un azéotrope avec l'alcool estérifiant à partir d'une colonne de distillation surmontant le réacteur d'estérification comprenant une résine cationique comme catalyseur, caractérisé en ce que le rapport molaire alcool/acide en entrée du réacteur est compris entre 1,4 et 3, et en ce que le mélange brut réactionnel circule dans une boucle de recirculation associant le réacteur et la colonne d'élimination de l'eau, à un taux de recirculation allant de 6 à 25, exprimé par le rapport massique entre le flux envoyé dans la boucle et le flux envoyé à un traitement de purification.

Le procédé de l'invention comporte en outre un traitement de purification du mélange brut réactionnel sortant de la boucle de recirculation comprenant au moins les étapes :

(i) on soumet le mélange brut réactionnel à une distillation dans une colonne d'étêtage permettant d'obtenir :

  ◦ en tête un flux composé essentiellement des réactifs non réagis ;
  ◦ en pied un flux comprenant l'ester recherché, des impuretés liées à l'acide et à l'alcool, et des sous-produits lourds ;

(ii) on soumet le flux de pied de la colonne d'étêtage à une colonne de rectification permettant de séparer :

  ◦ en tête l'ester recherché purifié ;
  ◦ en pied un flux contenant des impuretés liées à l'acide et à l'alcool et des sous-produits lourds, qui est concentré sur un évaporateur à film ou distillé dans une colonne d'équeutage afin de recycler à la colonne de rectification les composés légers présents, et d éliminer le résidu final de sous-produits lourds.

Selon un mode de réalisation de l'invention, les conditions de fonctionnement de la colonne d'étêtage sont adaptées pour entrainer au moins en partie les impuretés légères liées à l'acide ou à l'alcool.

Selon un mode de réalisation de l'invention, on soumet le flux de pied de la colonne d'étêtage à un lavage avec un flux aqueux avant de l'envoyer sur la colonne de rectification de l'étape (ii).

Selon un mode de réalisation de l'invention, l'étape (i) d'étêtage est précédée d'une étape de distillation du mélange brut réactionnel dans une colonne d'équeutage permettant de séparer une partie des sous-produits lourds.

## BREVE DESCRIPTION DES FIGURES

- La Figure 1 représente de manière schématique la section de réaction pour la mise en oeuvre du procédé selon l'invention.
- La Figure 2 représente de manière schématique la section de purification du procédé selon l'invention.
- La Figure 3 illustre un mode de réalisation de la section purification du procédé selon l'invention, incluant une étape de lavage du flux étêté.
- La Figure 4 illustre un mode de réalisation de la section purification du procédé selon l'invention, incluant une étape préalable d'équeutage du mélange brut réactionnel.

## EXPOSE DETAILLE DE L'INVENTION

L'invention est maintenant décrite plus en détail et de façon non limitative dans la description qui suit.

Dans la présente description, les termes « compris entre » ou « allant de » s'entendent bornes comprises.

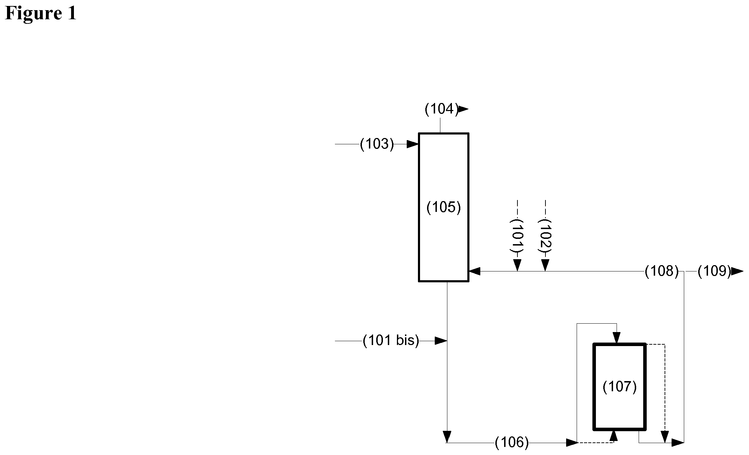

En faisant référence à la figure 1, de manière générale, la réaction d'estérification est effectuée dans un réacteur (107) surmonté d'une colonne de distillation (105) permettant d'extraire l'eau générée par la réaction. L'eau de réaction est éliminée au fur et à mesure de sa formation sous forme d'azéotrope avec l'alcool estérifiant dans le flux (104), afin de déplacer l'équilibre d'estérification.

Le réacteur peut être un réacteur à lit fixe ou un réacteur à lit en suspension.

L'ensemble représenté à la figure 1 forme une boucle de recirculation dans laquelle le mélange brut réactionnel effectue un certain nombre de passages dans le réacteur avant d'être envoyé à une section de purification. L'ensemble constitue ainsi un réacteur à boucle brassée avec élimination de l'eau. Il n'est pas nécessaire d'inclure une cuve agitée pour assurer le mélange des réactifs, ce mélange étant réalisé directement en ligne du fait de la présence de la boucle de recirculation.

Le réacteur peut être alimenté de manière descendante ou de manière ascendante, de préférence de manière descendante. L'alimentation en acide (méth)acrylique peut être réalisée à l'entrée de la colonne (flux 101) ou en pied de la colonne (flux 101 bis). L'alimentation en alcool (103) est réalisée directement dans la colonne de distillation. Un flux de recyclage (102) contenant de l'alcool et de l'acide non réagis, provenant de la section de purification peut être introduit également dans la boucle de recirculation, notamment en entrée de la colonne de distillation.

La colonne de distillation, en général à garnissage est équipée d'un condenseur de tête, un décanteur, recette et piège (non représentés sur la figure), permettant de décanter les vapeurs condensées en tête et de séparer une phase organique comprenant de l'alcool et des traces d'ester, qui est recyclée dans la colonne, et une phase aqueuse qui est éliminée. La colonne fonctionne généralement à une pression allant de 50 à 70 mm de Hg.

Selon l'invention, la réaction d'estérification est réalisée dans des conditions d'excès d'alcool, en particulier le rapport molaire alcool/acide à l'entrée du réacteur est compris entre 1,4 et 3, de préférence entre 1,8 et 2,3. Le rapport molaire alcool/acide fait référence aux teneurs en alcool et en acide des différents flux alimentant le réacteur d'estérification (flux de réactifs purs, flux recyclé et flux recirculé). Une analyse en ligne du flux (106) débarrassé de l'eau de la réaction entrant dans le réacteur permet d'ajuster les flux d'alimentation en réactifs purs pour obtenir le rapport souhaité.

Le réacteur comprend une résine cationique comme catalyseur d'estérification, de préférence une résine cationique forte, par exemple une résine cationique forte sulfonée de type styrène/divinyl benzène à groupements sulfoniques. A titre d'exemple de résines, on peut citer celles commercialisées sous les dénominations DIAION® PK208 ou PK216 par la société Mitsubishi, ou celles commercialisées sous la dénomination LEWATIT® K2620 ou K2621 par la société Lanxess, ou celles commercialisées sous la dénomination AMBERLYST® A15, A16 ou A46 par la Société Rohm & Haas.

La température de réaction est généralement comprise entre 70° et 100°C, de préférence entre 75°C et 95°C. La présence de la boucle de recirculation permet d'utiliser une température plus basse à production constante.

On conduit la réaction généralement en présence d'au moins un inhibiteur de polymérisation choisi parmi la phénothiazine, l'hydroquinone (HQ), et ses dérivés tels que l'éther méthylique de l'hydroquinone (EMHQ), le 2,6-diterbutyl-4-méthyl phénol (BHT), le 2,4-diméthyl-6-terbutyl phénol (Topanol A), les sels de l'acide thiocarbamique ou dithiocarbamique, les composés N-oxyls, comme le 4-hydroxy-2,2,6,6-tétraméthyl pipéridinoxyl (4-OH Tempo), les composés à groupements nitroso, tels que la N-nitroso phényl hydroxylamine et ses sels d'ammonium, les quinones telles que la benzoquinone, et les composés aminés comme les dérivés de paraphénylènediamine, à des teneurs dans le milieu de réaction pouvant être comprises entre 50 ppm et 5000 ppm, éventuellement en présence d'air appauvri, mais généralement à des teneurs comprises entre 150 ppm et 1000 ppm. L'ajout des inhibiteurs de polymérisation peut se faire à différents endroits, avec l'introduction des réactifs ou en tête de colonne de distillation.

Selon l'invention, le mélange brut réactionnel issu de la réaction est envoyé dans la boucle de recirculation, à un taux de recirculation allant de 6 à 25, ce taux étant exprimé par le rapport massique entre le flux envoyé dans la boucle et le flux envoyé au traitement de purification, en particulier par le rapport massique entre le débit de mélange brut réactionnel recirculant (108) et le débit de mélange brut réactionnel sortant de la boucle (109). De préférence, le taux de recirculation est compris entre 10 et 20, plus préférentiellement entre 10 et 15. Dans ces conditions, le temps de séjour global du mélange brut réactionnel au contact de la résine catalytique, exprimé par le rapport entre le volume de résine et le débit volumique total d'alimentation des réactifs est compris entre 2 heures et 6 heures, avantageusement entre 2,5 heures et 5 heures.

Le procédé selon l'invention s'applique à la synthèse de (méth)acrylates d'alkyle en $C_4$-$C_{10}$, l'alcool estérifiant étant un alcool aliphatique primaire ou secondaire, comportant une chaine alkyle linéaire ou ramifiée comportant de 4 à 10 atomes de carbone. Comme exemples d'alcools, on peut citer le butanol, le 2-éthyl hexanol, le n-octanol, le 2-octanol, le n-décanol et 2-propyl heptanol.

De préférence, l'alcool est le 2-éthyl hexanol ou le 2-octanol.

De préférence, on utilise l'acide acrylique.

Le procédé selon l'invention permet d'optimiser l'élimination de l'eau de réaction dans le flux entrant dans le réacteur, ce qui a avantageusement pour effet de limiter la formation d'impuretés acides liées à la présence de l'acide (méth)acrylique.

En effet le problème qui se pose avec la mise en oeuvre de l'acide acrylique est celui de la formation d'acide β-hydroxypropionique (dénommé dans la suite « AHP »), et d'acide β-acryloxypropionique (dénommé dans la suite « dimère » d'AA).

L'AHP se forme vraisemblablement à partir du dimère d'AA en présence d'eau et au contact de la résine. Sa formation dépend des conditions opératoires de réaction, de la nature de la résine cationique utilisée, et de la quantité d'eau présente.

En ce qui concerne les dimères d'AA, leur formation est pénalisante sur la quantité de sous-produits lourds à incinérer, et en conséquence pour la productivité.

Les mêmes types d'impuretés peuvent également se former dans le cas de l'acide méthacrylique, en particulier l'acide β-hydroxyméthylpropionique et l'acide β-méthacryloxypropionique.

Les conditions opératoires de l'invention minimisent la formation de ces impuretés et permettent d'obtenir, après la section de purification, l'ester recherché exempt d'impuretés liées à l'acide et conforme aux normes en matière de pureté pour préparer des polymères dans la plupart des domaines d'application notamment celui des adhésifs sensibles à la pression (PSA).

Au titre des autres réactions secondaires pouvant conduire à la formation de sous-produits lourds, l'alcool estérifiant et l'acide (méth)acrylique n'ayant pas encore réagi s'ajoutent à la double liaison de l'ester déjà formé (addition de Michael) pour former des dérivés (méthyl)propioniques et (méth)acryloxypropioniques dont le point d'ébullition se trouve au-dessus des points d'ébullition de l'acide (méth)acrylique, de l'alcool estérifiant et de l'ester formé.

Ces sous-produits lourds posent un problème de perte de matières premières et un problème de séparation et de traitement pour être finalement éliminés.

Il a été observé de façon surprenante que les conditions opératoires du procédé selon l'invention conduisent à une formation moindre de sous-produits lourds, il en résulte une simplification de la section de purification et une amélioration du bilan matières.

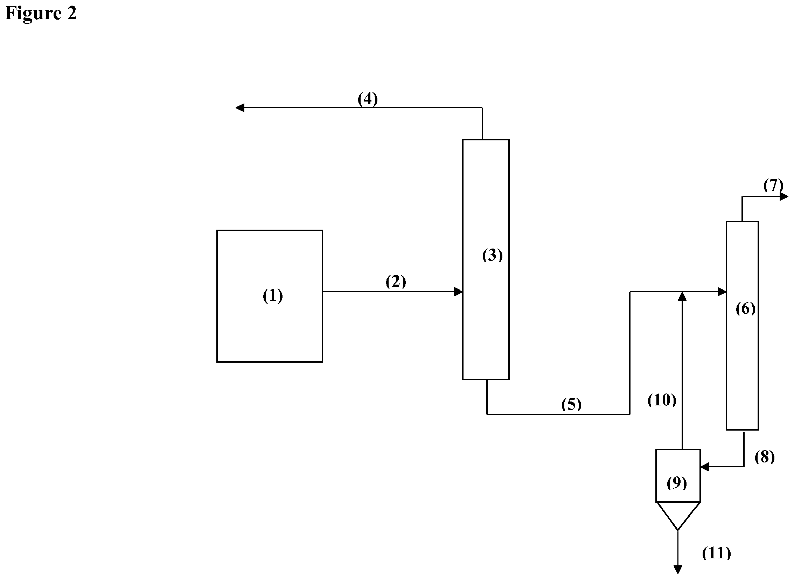

Après la zone de réaction, le mélange réactionnel sortant de la boucle de recirculation est soumis à un traitement de purification représenté par exemple sur la Figure 2 dans laquelle la boucle de recirculation est schématisée en (1).

Le mélange brut réactionnel (2) sortant de la boucle de recirculation est envoyé sur une colonne d'étêtage (3) qui sépare, en tête, un flux (4) comprenant essentiellement les réactifs non réagis, et en pied, un flux (5) comportant principalement l'ester recherché avec des impuretés liées à l'acide et à l'alcool et des sous-produits lourds. La colonne (3) est par exemple une colonne à plateaux, type plateaux perforés, ou à garnissage. Le flux (5) est envoyé sur une colonne de rectification (6) conduisant en tête à un flux (7) d'ester purifié, et en pied à un flux (8) qui est concentré sur un évaporateur à film (9) ou distillé dans une colonne d'étêtage (non représentée) afin de recycler les composés légers (10) présents vers le début de la section de purification, tels que des traces de réactifs non réagis, et d éliminer le résidu (11) final de produits lourds.

Le flux (4) comprend essentiellement les réactifs non réagis, acide (méth)acrylique et alcool estérifiant, qui sont séparés de l'ester recherché en raison de leur point d'ébullition plus faible. Ce flux (4), valorisable, est recyclé à la réaction. Dans le cas où des impuretés liées à l'acide sont formées dans la zone réactionnelle, il est possible d'adapter les conditions de fonctionnement de la colonne d'étêtage de façon à entraîner l'AHP formé (dans le cas de l'acide acrylique) dans le flux de tête recyclé à la réaction. L'AHP recyclé à la réaction peut réagir avec l'alcool estérifiant pour donner de l'hydroxypropionate d'alkyle, qui est facilement éliminé avec la fraction de sous-produits lourds en pied de la colonne de rectification. Ce mode de réalisation de l'invention contribue aussi à obtenir un produit final exempt d'acidité résiduelle.

Selon un mode de réalisation de l'invention, on effectue un lavage à l'eau du mélange réactionnel après étêtage, avant de séparer les impuretés et les sous-produits lourds dans la colonne de rectification.

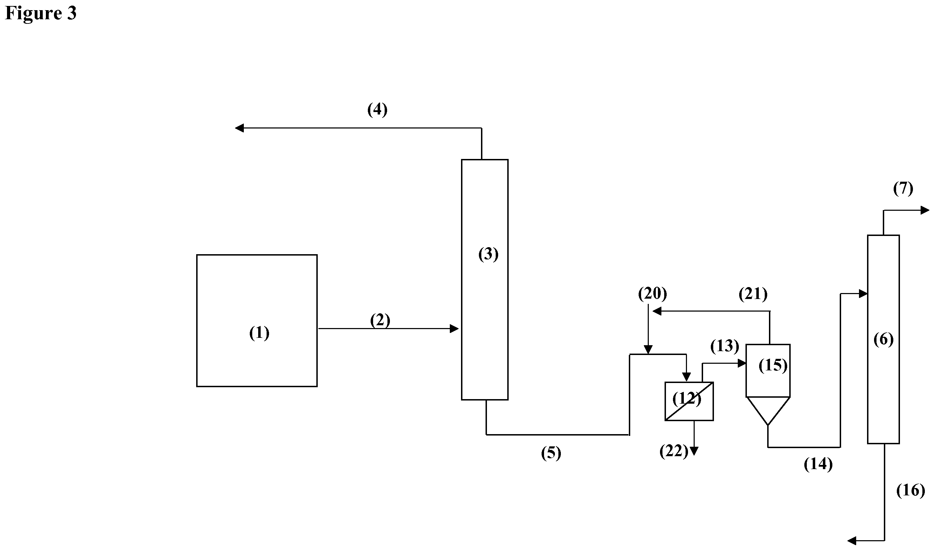

Selon ce mode de réalisation illustré à la Figure 3, le traitement de purification du mélange réactionnel sortant de la boucle de recirculation comprend au moins les étapes :

(i) on soumet le mélange brut réactionnel à une distillation dans une colonne d'étêtage permettant d'obtenir :

◦ en tête un flux composé essentiellement des réactifs non réagis ;
◦ en pied un flux comprenant l'ester recherché, des impuretés liées à l'acide et à l'alcool, et des sous-produits lourds ;

(i)a on soumet le flux de pied de la colonne d'étêtage à un lavage avec un flux aqueux permettant d'obtenir, après décantation,

◦ une phase aqueuse comprenant la totalité des impuretés liées à l'acide (méth)acrylique, et
◦ une phase organique comprenant l'ester recherché, des sous-produits lourds et des traces d'eau et d'acide (méth)acrylique ;

(ii) on soumet ladite phase organique, après élimination de l'eau, à une colonne de rectification permettant de séparer :

◦ en tête l'ester recherché purifié
◦ en pied un flux contenant des sous-produits lourds, qui est concentré sur un évaporateur à film ou distillé dans une colonne d'équeutage afin de recycler à la colonne de rectification les composés légers présents, et d éliminer le résidu final de sous-produits lourds.

En référence à la Figure 3, le flux de pied (5) de la colonne d'étêtage (3), contient en général plus de 90% en poids d'ester, voire plus de 94% en poids d'ester, des impuretés liées à l'acide et à l'alcool, et des sous-produits lourds, ainsi que les inhibiteurs de polymérisation.

Selon l'invention, l'étape d'extraction à l'eau est effectuée avec un flux aqueux (20), qui comprend de l'eau pure, ou de l'eau issue de l'étape de réaction, ou un mélange des deux en toutes proportions.

La température de lavage n'est pas critique, elle est en général comprise entre 25°C et 100°C, préférentiellement entre 50°C et 70°C.

On utilise de l'ordre de 5g à 10g d'eau pour 100g de flux (5), afin de l'en débarrasser de la totalité des impuretés liées à l'acide qui risqueraient de catalyser la décomposition des composés lourds dans la suite du processus de purification. Il n'est pas nécessaire d'utiliser plus d'eau pour éliminer la totalité des impuretés acides.

Un lavage avec 10% d'eau permet, par exemple, de faire passer une teneur en AHP de l'ordre de 120 ppm à moins de 1 ppm et de réduire de 15 à 20% la teneur en dimère d'AA.

La quantité d'eau de lavage est de préférence optimisée en fonction de la teneur en dimère d'AA présente dans le flux (5).

La phase aqueuse (22) contient l'essentiel des impuretés liées à l'acide et un peu d'acide.

La phase organique décantée (13) dans le décanteur (12) contient l'ester et une fraction minoritaire de composés lourds et de composés légers avec de l'eau résiduelle, de l'ordre de 3500 ppm à 5000 ppm d'eau qu'il est préférable d'éliminer avant de soumettre ladite phase à la distillation finale.

La phase organique obtenue après lavage à l'eau est donc soumise à une étape d'élimination d'eau, l'eau récupérée pouvant être recyclée à l'étape de lavage.

L'eau présente dans ladite phase organique (13) peut être éliminée par distillation à l'aide d'une colonne à distiller ou à l'aide d'un évaporateur à film mince (15). Dans un mode préféré de l'invention, on utilise un évaporateur à film mince type Luwa. Les conditions opératoires, données uniquement à titre indicatif sont dans ce cas, une pression de 150mbar, une température tête évaporateur de 24°C, une température huile de 130°C, une répartition massique tête/pied évaporateur 10/90.

Le passage sur l'évaporateur (ou colonne à distiller) permet de réduire la teneur en eau à plus de 95% dans le flux (14) et de générer un distillat aqueux (21), pouvant contenir un peu d'ester qui est, tout ou partie, recyclé à l'étape de lavage.

Le flux aqueux (20) utilisé pour le lavage est ainsi préférentiellement un mélange du flux aqueux (21) provenant du procédé avec un appoint d'eau fraîche.

Le flux exempt d'eau (14) est ensuite envoyé vers la dernière colonne de rectification (6) de l'ester pur. La colonne (6) est par exemple une colonne à plateaux, type plateaux perforés, ou à garnissage. Du fait de l'élimination de l'essentiel du dimère d'acide lors de l'étape de lavage à l'eau, les conditions de fonctionnement de la colonne (6) sont facilitées et la décomposition des composés lourds est minimisée.

Le produit distillé en tête de colonne (6) est un ester purifié ne contenant plus d'impuretés tel que l'AHP, et contenant une teneur en général inférieure à 500 ppm en alcool estérifiant et une teneur en dimère d'acide en général inférieure à 100 ppm.

Le flux (16) en pied de colonne (6), riche en composés lourds et pouvant contenir des traces d'ester peut être recyclé partiellement ou éliminé.

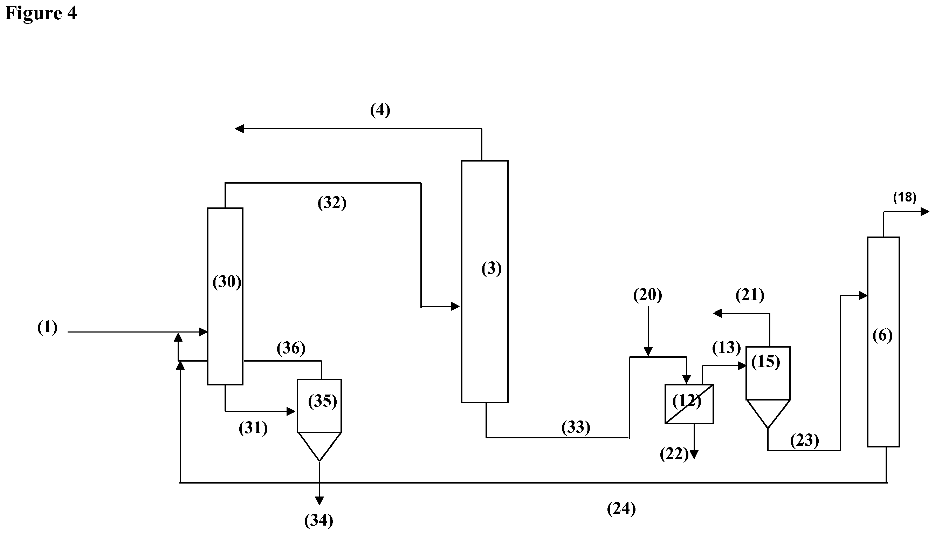

Selon un autre mode de réalisation de l'invention illustré à la Figure 4, l'étape (i) d'étêtage est précédée d'une étape de distillation du mélange réactionnel dans une colonne d'équeutage permettant de séparer en pied un flux comprenant l'essentiel du dimère d'acide et des composés lourds, avec des traces d'ester, et en tête un flux comprenant l'ester, les réactifs non réagis et des impuretés liées à l'acide et des traces de composés lourds, ledit flux de tête étant alors soumis à l'étape (i) d'étêtage.

Selon ce mode de réalisation de l'invention, le flux de pied de la colonne d'étêtage dans l'étape (i), ainsi que la phase organique lavée dans l'étape (i)a contient des impuretés liées à l'acide et des composés lourds en quantité réduite, ce qui permet de réduire la quantité de flux en pied de la dernière colonne de distillation et d'obtenir un produit pur sans acidité résiduel et exempt d'alcool résiduel.

En référence à la Figure 4, la colonne d'étêtage (3) est précédée d'une colonne d'équeutage (30), du type colonne à plateaux ou à garnissage.

Le mélange réactionnel (1) est envoyé sur une première colonne de distillation (30) afin d'en séparer l'essentiel du dimère d'acide et des composés lourds (équeutage) et soumettre à l'étape d'étêtage un flux appauvri en dimère d'acide et composés lourds.

Le flux de pied (31) de la colonne (30) contient, outre du dimère d'acide et des composés lourds, avec des traces de composés légers et d'ester.

Ce flux (31) est avantageusement concentré, par exemple sur un évaporateur à film (35) permettant de recycler les composés légers avec l'ester à l'alimentation de la colonne (30), et d'éliminer les résidus lourds (34), par exemple par incinération.

Le flux de tête (32) de la colonne (30), débarrassé de la majorité des composés lourds et de la quasi totalité des dimères d'acide est ensuite envoyé sur la colonne d'étêtage (3), d'où sort en tête un flux (4) contenant les produits légers avec des traces d'ester. Le flux de pied (33) de la colonne d'étêtage (3) comprend plus de 90% en poids, voire plus de 94% en poids d'ester, des impuretés liées à l'acide et des composés lourds non séparés lors de l'étape d'équeutage, et des traces de composés légers.

Comme dans le mode de réalisation décrit précédemment, ce flux (33) est traité avec un flux aqueux (20) pour l'en débarrasser des impuretés liées à l'acide.

Une quantité d'eau de l'ordre de 10% par rapport au flux à traiter permet en général d'éliminer la totalité des impuretés liées à l'acide dans la phase aqueuse, et une partie du dimère d'acide dont la teneur est déjà réduite du fait de l'étape préalable d'équeutage.

La phase aqueuse de décantation (22) contenant les impuretés liées à l'acide est éliminée ou recyclée. La phase organique (13) est envoyée vers le sécheur (15) qui peut être constitué d'une colonne ou préférentiellement d'un évaporateur à film.

Le flux (21) contenant de l'eau et de l'ester est, tout ou partie, recyclé pour effectuer le lavage du flux (33), minimisant ainsi la teneur en eau fraiche (20) à introduire dans le circuit de lavage.

Le flux séché (23), est envoyé sur une dernière colonne de rectification (6), d'où sort en tête l'ester purifié (18). En pied, le flux (24) contenant de l'ester, et des traces d'alcool est avantageusement recyclé à l'alimentation de la colonne (30).

Selon ce mode de réalisation de l'invention, on obtient un ester exempt d'acidité résiduelle et contenant une teneur en alcool en général inférieure à 200 ppm et une teneur en dimère d'acide en général inférieure à 5 ppm.

La mise en oeuvre d'une étape de séparation des composés lourds dans la colonne d'équeutage (30) en amont de la séparation des composés légers dans la colonne d'étêtage (3) permet de véhiculer dans le reste du train de distillation un flux quasi exempt de dimère d'acide et de composés lourds.

L'absence de ces impuretés dans le flux (23) alimentant la colonne de rectification (6) du produit pur permet de faciliter le fonctionnement de cette colonne de distillation, de réduire le taux de reflux et la puissance de chauffe sur la colonne, ce qui conduit à une meilleure productivité de l'installation, une production réduite de flux de pied (24), et à la quasi absence de dimère d'acide dans l'ester pur. La teneur en alcool résiduel dans l'ester pur s'en trouve également réduite en raison de la quasi absence de composés lourds et d'impuretés liées à l'acide dans l'alimentation de la dernière colonne de distillation.

Les exemples ci-après illustrent la présente invention sans toutefois en limiter la portée.

PARTIE EXPERIMENTALE

Dans les exemples, les pourcentages sont indiqués en poids sauf indication contraire et les abréviations suivantes ont été utilisées :

    AA : acide acrylique
    A2EH : acrylate de 2-éthylhexyle
    2EH : 2-éthyl hexanol
    AHP : acide β-hydroxypropionique
    Di-AA : dimères d'AA
    PTZ : phénothiazine

**Exemple 1 :** influence du rapport molaire alcool/acide (RM)

L'appareillage utilisé pour la synthèse d'acrylate de 2-éthylhexyle est un micropilote de synthèse reproduisant le fonctionnement d'un réacteur à boucle brassée avec élimination de l'eau, tel que représenté à la Figure 1.

Le réacteur contient 340 ml (volume apparent initial) de résine cationique K2620 de Lanxess.

Le réacteur est alimenté en acide acrylique et en 2-éthyl hexanol purs. Aucun flux recyclé n'est introduit.

Pour chaque passage, le temps de séjour du mélange réactionnel sur la résine, représenté par le rapport entre le volume de résine et le débit volumique du flux recirculé dans la boucle est égal à 18 mn. Le temps de séjour global, représenté par le rapport entre le volume de résine et le débit volumique du flux d'alimentation est égal à 2,9 heures.

La colonne de distillation est une colonne de laboratoire de type vigreux correspondant à environ 6 étages théoriques et fonctionne à une pression de 80 mm Hg, avec une température de tête de 35°C. L'eau de réaction est éliminée sous forme d'un azéotrope avec le 2-éthylhexanol.

Deux séries d'essai ont été réalisées, la première série à une température du lit de résine de 87°C, la seconde série à une température de 95°C.

Le taux de recirculation dans la boucle de recirculation est égal à 7,9 ou 8.

Pour chaque série, le débit d'alimentation en alcool et en acide a été ajusté de façon à ce que le rapport molaire en entrée du réacteur soit égal à 1,1 (ou 1,2) et 2 (2,2).

Pour chaque condition de test, 2 à 3 prélèvements de mélange réactionnel ont été réalisés après au minimum 48 h de fonctionnement. Les échantillons prélevés ont été analysés par chromatographie en phase gazeuse ou liquide, de façon à déterminer leur composition massique, et calculer les taux de conversion et les sélectivités selon les formules suivantes :

$$\text{Taux de conversion en acide, } \% = \text{ moles d'acide converti / moles d'acide en alimentation x } 100$$

Sélectivité de l'ester par rapport à l'acide, % = moles d'ester formé /moles

d'acide converti x 100

Sélectivité de l'ester par rapport à l'alcool, % = moles d'ester formé /moles

d'alcool converti x 100

Avec le nombre de moles d'acide converti calculé à partir de la différence entre le nombre de moles d'acide en alimentation et le nombre de moles d'acide sortant de la boucle de recirculation (dans la phase aqueuse en tête colonne et dans le mélange réactionnel brut sortant) ;
Avec le nombre de moles d'alcool converti calculé à partir de la différence entre le nombre de moles d'alcool en alimentation et le nombre de moles d'alcool sortant de la boucle de recirculation (dans le mélange réactionnel brut sortant)

Un bilan est réalisé aux bornes de la boucle complète en quantifiant les principaux sous-produits.
Les résultats sont rassemblés dans le tableau 1.

Tableau 1

|  | Essai 1 comparatif | Essai 2 | Essai 3 comparatif | Essai 4 |
|---|---|---|---|---|
| Température, °C | 87 | 87 | 95 | 95 |
| Rapport molaire 2EH/AA entrée du réacteur | 1,1 | 2 | 1,2 | 2,2 |
| Taux de recirculation | 7,9 | 7,9 | 8 | 8 |
| Conversion AA, % | 66,0 | 71,5 | 73,8 | 79,3 |
|  | Essai 1 comparatif | Essai 2 | Essai 3 comparatif | Essai 4 |
| Sélectivité ester / AA converti, % | 98,7 | 99,9 | 97,5 | 99,8 |
| Sélectivité ester / 2EH converti, % | 100,2 | 100,1 | 97,9 | 97,4 |
| Bilan sous-produits lourds / ester formé, g/kg | 43,1 | 34,3 | 60,0 | 46,6 |
| Di-AA / ester formé, g/kg | 1,3 | 0,6 | 2,0 | 0,7 |
| AHP / ester formé, g/kg | 0,31 | 0,19 | 0,48 | 0,31 |

Ces essais montrent qu'une augmentation du rapport molaire alcool/acide à l'entrée du réacteur permet d'améliorer la conversion de l'acide (la concentration d'AA en entrée étant plus faible), mais également la sélectivité de l'ester par rapport à l'acide converti, pour une sélectivité de l'ester par rapport à l'alcool sensiblement constante.
Le rapport molaire alcool/acide à l'entrée du réacteur a un effet sur la production de sous-produits lourds, selon l'invention, la quantité de sous-produits générés lors de la réaction diminue de plus de 20%, ce qui a un impact bénéfique pour la purification de l'ester et sur la quantité de matières éliminées. Cet effet est accentué à la température plus élevée.
Les impuretés liées à l'acide sont générées en quantité réduite dans les conditions de l'invention.

Exemple 2 : influence du rapport molaire alcool/acide (RM)

Cet exemple illustre l'effet du RM sur une unité industrielle de production d'acrylate de 2-éthylhexyle à une échelle de 90 tonnes/jour.
L'unité a fonctionné avec un rapport molaire alcool/acide en entrée d'un lit catalytique allant de 1,25 à 1,9 (3 productions P1, P2 et P3). La température a été maintenue constante à 85°C, ainsi que le taux de recirculation est resté sensiblement identique.
Le réacteur est un lit fixe de 40 $m^3$ d'une résine cationique forte de type macroporeuse. Il est alimenté dans le sens descendant.
Les résultats sont rassemblés dans le tableau 2.

Tableau 2

|  | P1 comparatif | P2 | P3 |
|---|---|---|---|
| Température, °C | 85 | 85 | 85 |
| RM | 1,25 | 1,6 | 1,9 |
| Taux de recirculation | 10,5 | 11 | 12 |
| Taux de conversion AA global % | 69,1 | 69,4 | 71,3 |
| Sélectivité ester / AA converti, % | 89,1 | 93,5 | 95,8 |
| Sélectivité ester / 2EH converti, % | 91,1 | 94,5 | 95,9 |
| Di-AA dans le produit brut, ppm | 325 | 240 | 149 |
| AHP dans le produit brut, ppm | 75 | 75 | 61 |
| Sous-produits lourds dans le produit brut, ppm | 17900 | 12800 | 11600 |

La production industrielle réalisée dans les conditions de RM selon l'invention a permis d'améliorer sensiblement la sélectivité de l'ester par rapport à l'acide et l'alcool et d'obtenir un produit brut d'acrylate de 2-éthylhexyle contenant seulement des traces d'impuretés liées à l'acide. Le procédé a généré en outre moins de sous-produits lourds.

**Exemple 3 :** Effet de l'eau sur la formation des impuretés liées à l'acide et des sous-produits lourds

Cet exemple a pour but de montrer l'effet néfaste de la présence d'eau dans le réacteur sur la formation d'AHP et de dimère d'AA, et sur la quantité globale de sous-produits lourds.
L'appareillage utilisé est celui de l'exemple 1 avec les conditions opératoires suivantes : Résine A16 de DOW.

Température de réaction : 88°C
Débit d'alimentation d'AA frais : 39 g/h
Débit du flux recirculant dans la boucle : 950 g/h
Taux de recirculation : 8
Rapport molaire alcool/acide dans l'alimentation : 1,1
Rapport molaire alcool/acide entrant dans le réacteur : 1,2-1,3
Temps de séjour dans le réacteur pour un passage : 20,6 mn
Temps de séjour global dans le réacteur : 3,6 heures

L'essai A a été effectué en utilisant de l'AA pur, et l'essai B avec un flux d'AA additivé de 1% massique d'eau.
Les résultats du tableau 3 montrent que la quantité des impuretés liées à l'acide et des sous-produits lourds est plus importante dans l'essai B comparativement à l'essai A.

Tableau 3

|  | Essai A | Essai B |
|---|---|---|
| Alimentation en acide | AA pur | AA + 1% d'eau |
| Conversion AA, % en une passe | 15,8 | 7,6 |
| Conversion globale AA, % | 68,1 | 62,5 |
| Sélectivité ester / AA converti, % | 40,2 | 42,4 |
| Sélectivité ester / 2EH converti, % | 72,4 | 74,3 |
| Bilan sous-produits lourds / ester formé, g/kg | 41,9 | 54,5 |
| Di-AA/ ester formé, g/kg | 1,2 | 2,3 |
| AHP / ester formé, g/kg | 0,34 | 0,79 |

**Exemple 4 :** influence du taux de recirculation

Deux séries d'essais ont été réalisées sur une unité industrielle en faisant varier le taux de recirculation dans la boucle de recirculation.

De la même façon, la conversion en acide, les sélectivités en ester et la quantité de sous-produits lourds ont été déterminées à partir de prélèvements effectués après au minimum 48 h de fonctionnement.

Les résultats sont rassemblés dans le tableau 4.

Tableau 4

|  | Essai (a) | Essai (b) |
|---|---|---|
| Température, °C | 79 | 79 |
| Rapport molaire 2EH/AA entrée du réacteur | 2,3 | 2,3 |
| Taux de recirculation | 24 | 15 |
| Conversion AA, % | 75-80 | 68-72 |
| Bilan sous-produits lourds / ester formé, kg/t | 22 | 26 |
| Di-AA / ester formé, kg/t | 0,001 | 0,001 |
| AHP / ester formé, kg/t | 0,02 | 0,11 |

Le taux de conversion de l'acide acrylique augmente lorsque le taux de recirculation augmente. La quantité de sous-produits lourds rapportée à la production d'ester diminue lorsque le taux de recirculation augmente.

Le mélange brut réactionnel obtenu à l'essai (a) a été soumis à une section de purification telle que représentée à la Figure 2. En particulier la colonne d'étêtage a été réglée de façon à séparer la majorité de l'AHP en tête de colonne et obtenir un ester sensiblement exempt d'impuretés liées à l'acide.

Le tableau 5 rassemble la composition en % massiques des différents flux : flux (2) en sortie de la boucle réactionnelle, flux (5) en pied de la colonne d'étêtage, flux (4) en tête de la colonne d'étêtage, le flux d'ester purifié (7).

Tableau 5

| % massiques | Flux (2) | Flux (5) | Flux (4) | Flux (7) |
|---|---|---|---|---|
| A2EH | 61,2 | 97,4 | 26,1 | 99,79 |
| AA | 5,8 | < 0,001 | 11,9 | 0,0007 |
| di-AA | 0,0068 | 0,032 | - | 0,0002 |
| AHP | 0,0029 | 0,0017 | 0,0042 | 0,0023 |
| Produits lourds | 4,9 | 2,6 |  |  |
| Composés légers |  |  | 7,4 | - |

**Exemple 5** (en référence à la Figure 3)

Un mélange brut réactionnel (1) obtenu selon le procédé de l'invention a la composition massique suivante :

- A2EH : 67,8%
- 2EH : 19,4%
- AA : 7,7%
- AHP : 119 ppm
- Dimère d'AA : 780 ppm
- composés lourds : qsp 100%

Le mélange brut réactionnel est envoyé sur une colonne d'étêtage (3) qui sépare, en tête, un flux (4) comprenant essentiellement des composés légers, notamment les réactifs résiduels AA et 2EH, de l'eau, des octènes, un peu d'acétate de 2-éthylhexyle et des traces d'A2EH, et, en pied, un flux (5) comportant principalement l'A2EH recherché avec des impuretés dont les sous-produits lourds.

Le flux (4) est recyclé à la réaction.

Le flux (5) en pied de la colonne d'étêtage (3) a la composition suivante :

- A2EH : 94,35%
- 2EH : 0,041%
- AA : 17 ppm
- AHP : 32 ppm
- Dimère d'AA : 1340 ppm

On lave le flux (5) pendant 10 min à 70°C avec de l'eau pure (20), à raison de 10g d'eau pour 100g de flux (5), dans une capacité agitée chauffée par circulation d'huile dans une double enveloppe (non représenté), puis le flux lavé est décanté pendant 30 min à 70°C dans le décanteur (12).

La phase organique décantée (13) a la composition massique suivante :

- A2EH : 94,22%
- 2EH : 580ppm
- eau : 3800 ppm
- AA : 15 ppm
- Dimère d'AA : 1070 ppm
- AHP : néant
- composés lourds : qsp 100%

On observe que :

19% du Dimère d'AA est passé dans la phase aqueuse décantée (22) ;
100% de l'AHP est extrait dans la phase aqueuse ;
10% de l'acidité totale (AHP + AA+ dimère d'AA) est passée en phase aqueuse.

La phase organique (13) est envoyée sur un évaporateur à film mince Luwa (15), afin d'en éliminer l'eau présente, sous les conditions suivantes : T° huile 130°C - P : 150 mbar.

La teneur résiduelle en eau dans la phase organique séchée (14) est de 100 ppm.

La composition massique du flux (14) est la suivante :

- A2EH : 94,32%
- 2EH : 400 ppm
- AA : 16 ppm
- AHP : néant
- Dimère d'AA : 1105 ppm
- eau : 100 ppm

Ce flux (14) est distillé en continu sur une colonne à garnissage (6) ayant une efficacité d'environ 6 plateaux théoriques, avec les conditions :

Pression tête : 39 mbar - T° tête colonne : 107°C - T° pied : 120°C

Le flux (7) d'A2EH purifié, distillé en tête de la colonne a la composition suivante :

- A2EH : 99,64%
- 2EH : 474 ppm
- AA : 18 ppm
- AHP : néant
- Dimère d'AA : 60 ppm

On obtient un acrylate de 2-éthylhexyle, exempt d'AHP et appauvri en 2éthyl hexanol résiduel.

## Exemple 6

L'exemple 5 est repris en réutilisant 3 fois consécutivement la phase aqueuse (22) pour effectuer le lavage du flux (5) (non représenté sur la figure).

Le flux (5) est lavé dans les conditions de l'exemple 2 puis la phase aqueuse décantée ($\phi A_1$) est réutilisée pour laver le flux (5).
La phase aqueuse décantée ($\phi A_2$) est utilisée pour laver une troisième fois le flux (5). La phase aqueuse décantée notée ($\phi A_3$).

|  | (22) | $\phi A_1$ | $\phi O_1$ | $\phi A_2$ | $\phi O_2$ | $\phi A_3$ | $\phi O_3$ |
|---|---|---|---|---|---|---|---|
| AHP, ppm | 30 | 287 | néant | 563 | néant | 832 | néant |
| Dimère d'AA,ppm | 1332 | 2003 | 1068 | 2357 | 1110 | 2386 | 1200 |
| AA ppm | 142 | 28 | 30 | 32 | 33 | 37 | 34 |

Cet exemple montre que l'eau de lavage peut être réutilisée plusieurs fois sans perte d'efficacité sur l'élimination de l'AHP, et ainsi de réduire la consommation d'eau pure dans l'étape de lavage.

**Exemple 7** (en référence à la Figure 4)

Un brut réactionnel (1) a la composition massique suivante :

- A2EH : 66,26%
- 2EH : 20,64%
- AA : 8,56%
- AHP : 130 ppm
- Dimère d'AA : 684 ppm
- Composés lourds : qsp 100%

Les principaux sous-produits lourds présents dans le brut (1) sont :

- hydroxypropionate de 2-éthylhexyle : 0,54% - acryloxypropionate de 2-éthylhexyle : 0,87% - 2-éthylhexyl-oxypropionate de 2-éthylhexyle : 0,42%.

La totalité des sous-produits lourds présents dans le brut réactionnel (1) représente 67,5g/h.
Le brut réactionnel (1) est envoyé, en continu, sur une colonne de Vigreux (30) ayant une efficacité théorique d'environ 5 plateaux théoriques à un débit de 3245g/h pour séparer l'essentiel des sous-produits lourds (équeutage). Les conditions sont les suivantes :

Pression en tête de colonne : 47 mbar ;
Température en tête de colonne :100-108°C ;
Température en pied de colonne : 115-120°C.
Débit de flux de tête (32) : 2953g/h
Débit du flux de pied (31) : 291,8g/h

La composition massique du flux de tête (32) de la colonne d'équeutage (30) est :

- A2EH : 65,46%
- 2EH : 22,92%
- AA : 9,66%
- AHP : 135 ppm
- Dimère d'AA : 14 ppm
- composés lourds : néant

La composition massique du flux de pied (31) de la colonne d'équeutage (30) est :

- A2EH : 60,43%
- 2EH : 0,053%
- AA : 11ppm
- Dimère d'AA : 6340 ppm
- Composés lourds : 17,5% (soit 51,2g/h)

La quasi totalité des composés lourds, ainsi que 97% du dimère d'AA sont séparés du brut réactionnel (1) et restent en pied de la colonne d'équeutage (30).

Le flux de tête (32) est exempt de composés lourds et de dimère d'AA, mais contient la quasi-totalité de l'AHP.

Le flux de pied (31) est traité sur un évaporateur à film mince Luwa (35), dans les conditions suivantes :

T°huile : 162°C - Pression : 65 mbar - débit d'alimentation : 270 g/h.

86 % des composés lourds restent en pied de l'évaporateur (35) et sont éliminés dans le flux (34).

En tête de l'évaporateur (35), sort un flux (36) qui contient 70% de l'A2EH, 86% du 2EH et 39% du dimère d'AA contenu dans le flux (31) ; le flux (36) est recyclé dans la colonne d'équeutage (30).

Le flux de tête (32) de la colonne d'équeutage (30) est envoyé sur la colonne d'étêtage (3).

La colonne d'étêtage (3) est du même type que celle présente dans les installations des figures 2 et 3.

Les conditions de fonctionnement de la colonne (3) sont les suivantes :

Pression de tête : 47 mbar
Température de tête : 105°C - température de pied : 143-148°C
Débit d'alimentation du flux (32) : 2234g/h
Débit du flux de tête (4) : 1203 g/h
Débit du flux de pied (33) : 1030,9 g/h

La composition massique du flux de tête (4) de la colonne d'étêtage (3) est :

-    A2EH : 40,44%
-    2EH : 40,06%
-    AA: 17,01%
-    AHP : 174 ppm
-    Dimère d'AA : 26 ppm
-    Composés lourds : néant

Le flux de tête (4) de la colonne d'étêtage (3) est renvoyé à la réaction.

La composition massique du flux de pied (33) de la colonne d'étêtage (3) est :

-    A2EH : 99,49%
-    2EH : 0,03%
-    AA : <1 ppm
-    AHP : 24 ppm
-    Dimère d'AA : 90 ppm
-    Composés lourds : 0,03%

Le flux de pied (33) de la colonne d'étêtage (3) est lavé avec un flux d'eau (20) à 70°C pendant 10 min, (92,2g d'eau pour 922,2g de flux (33)), puis décanté à 70°C pendant 30 min dans le décanteur (12).

On récupère 915,7g de phase organique décantée (13) et 94,7g de phase aqueuse (22). La phase organique (13) est séchée sur un évaporateur à film mince Luwa (15), afin d'en éliminer l'eau présente, opérant dans les conditions suivantes : T° huile : 100°C - Pression : 38 mbar.

La composition massique du flux séché (23) est :

-    A2EH : 99,45%
-    2EH : 0,03%
-    AA< 1 ppm
-    AHP : néant
-    Dimère d'AA : 77 ppm
-    eau < 100 ppm
-    Composés lourds : 0,03%

Le flux (23) est envoyé sur la dernière colonne de distillation (6), identique à celle des exemples let 2, séparant en tête un flux (18) d'A2EH pur.

Les conditions de fonctionnement de la colonne (6) sont :

Pression tête : 39 mbar - T° tête colonne : 107°C - T° pied : 120°C

Le flux (18) de tête a la composition suivante :

- A2EH : 99,72%
- 2EH : 195 ppm
- AA : 8 ppm
- AHP : néant
- Dimère d'AA : <1 ppm
- Composés lourds : néant

On obtient un acrylate de 2 éthylhexyle exempt d'AHP et contenant une teneur résiduelle en 2-éthyl hexanol très faible. Le flux (24) en pied de la colonne de distillation du pur est renvoyé à l'alimentation de la colonne d'équeutage (30).

**Revendications**

1.  Procédé de fabrication d'un (méth)acrylate d'alkyle en $C_4$-$C_{10}$, par estérification directe d'acide (méth)acrylique par l'alcool correspondant, l'eau de réaction étant éliminée sous la forme d'un azéotrope avec l'alcool estérifiant à partir d'une colonne de distillation surmontant le réacteur d'estérification comprenant une résine cationique comme catalyseur, **caractérisé en ce que** le rapport molaire alcool/acide en entrée du réacteur est compris entre 1,4 et 3, et **en ce que** le mélange brut réactionnel circule dans une boucle de recirculation associant le réacteur et la colonne d'élimination de l'eau, à un taux de recirculation allant de 6 à 25, exprimé par le rapport massique entre le flux envoyé dans la boucle et le flux envoyé à un traitement de purification.

2.  Procédé selon la revendication 1 **caractérisé en ce que** le rapport molaire alcool/acide en entrée du réacteur est compris entre 1,8 et 2,3.

3.  Procédé selon la revendication 1 ou 2 **caractérisé en ce que** le taux de recirculation est compris entre 10 et 20, de préférence entre 10 et 15, exprimé par le rapport massique entre le flux envoyé dans la boucle et le flux envoyé à un traitement de purification.

4.  Procédé selon l'une quelconque des revendications précédentes **caractérisé en ce que** l'acide est l'acide acrylique.

5.  Procédé selon l'une quelconque des revendications précédentes **caractérisé en ce que** l'alcool est le butanol, le 2-éthyl hexanol, le n-octanol, le 2-octanol, le n-décanol et 2-propyl heptanol, de préférence, le 2-éthyl hexanol ou le 2-octanol.

6.  Procédé selon l'une quelconque des revendications précédentes **caractérisé en ce que** le temps de séjour global du mélange brut réactionnel au contact de la résine catalytique, exprimé par le rapport entre le volume de résine et le débit volumique total d'alimentation des réactifs est compris entre 2 heures et 6 heures, de préférence entre 2,5 heures et 5 heures.

7.  Procédé selon l'une quelconque des revendications précédentes **caractérisé en ce qu'**il comporte en outre un traitement de purification du mélange brut réactionnel sortant de la boucle de recirculation comprenant au moins les étapes :

    (i) on soumet le mélange brut réactionnel à une distillation dans une colonne d'étêtage permettant d'obtenir :

    ◦ en tête un flux composé essentiellement des réactifs non réagis ;
    ◦ en pied un flux comprenant l'ester recherché, des impuretés liées à l'acide et à l'alcool, et des sous-produits lourds ;

    (ii) on soumet le flux de pied de la colonne d'étêtage à une colonne de rectification permettant de séparer :

    ◦ en tête l'ester recherché purifié ;
    ◦ en pied un flux contenant des impuretés liées à l'acide et à l'alcool et des sous-produits lourds, qui est concentré sur un évaporateur à film ou distillé dans une colonne d'équeutage afin de recycler à la colonne de rectification les composés légers présents, et d éliminer le résidu final de sous-produits lourds.

8.  Procédé selon la revendication 7 **caractérisé en ce que** les conditions de fonctionnement de la colonne d'étêtage sont adaptées pour entrainer au moins en partie les impuretés liées à l'acide.

**9.** Procédé selon la revendication 7 ou 8 **caractérisé en ce que** l'on soumet le flux de pied de la colonne d'étêtage à une étape de lavage (i)a avec un flux aqueux avant de l'envoyer sur la colonne de rectification de l'étape (ii).

**10.** Procédé selon la revendication 9 **caractérisé en ce que** l'étape de lavage (i)a permet d'obtenir, après décantation,

∘ une phase aqueuse comprenant la totalité des impuretés liées à l'acide (méth)acrylique, et
∘ une phase organique comprenant l'ester recherché, des sous-produits lourds et des traces d'eau et d'acide (méth)acrylique ;

la dite phase organique, après élimination de l'eau, étant soumise à l'étape (ii).

**11.** Procédé selon l'une quelconque des revendications précédentes **caractérisé en ce que** l'étape (i) d'étêtage est précédée d'une étape de distillation du mélange brut réactionnel dans une colonne d'équeutage permettant de séparer une partie des sous-produits lourds.

## Patentansprüche

**1.** Verfahren zur Herstellung eines $C_4$-$C_{10}$-Alkyl(meth)-acrylats durch direkte Veresterung von (Meth)acrylsäure mit dem entsprechenden Alkohol, wobei das Reaktionswasser in Form eines Azeotrops mit dem veresternden Alkohol aus einer über dem Veresterungsreaktor, der ein kationisches Harz als Katalysator enthält, angeordneten Destillationssäule entfernt wird, **dadurch gekennzeichnet, dass** das Alkohol/Säure-Molverhältnis am Eingang des Reaktors zwischen 1,4 und 3 liegt und dass die rohe Reaktionsmischung in einer Rezirkulationsschleife, die den Reaktor und die Säule zur Entfernung des Wassers miteinander verbindet, mit einer Rezirkulationsrate im Bereich von 6 bis 25, ausgedrückt durch das Massenverhältnis zwischen dem der Schlaufe zugeführten Strom und dem einer Reinigungsbehandlung zugeführten Strom, zirkuliert.

**2.** Verfahren nach Anspruch 1, **dadurch gekennzeichnet, dass** das Alkohol/Säure-Molverhältnis am Eingang des Reaktors zwischen 1,8 und 2,3 liegt.

**3.** Verfahren nach Anspruch 1 oder 2, **dadurch gekennzeichnet, dass** die Rezirkulationsrate zwischen 10 und 20, vorzugsweise zwischen 10 und 15, ausgedrückt durch das Massenverhältnis zwischen dem der Schlaufe zugeführten Strom und dem einer Reinigungsbehandlung zugeführten Strom, liegt.

**4.** Verfahren nach einem der vorhergehenden Ansprüche, **dadurch gekennzeichnet, dass** sich bei der Säure um Acrylsäure handelt.

**5.** Verfahren nach einem der vorhergehenden Ansprüche, **dadurch gekennzeichnet, dass** es sich bei dem Alkohol um Butanol, 2-Ethylhexanol, n-Octanol, 2-Octanol, n-Decanol und 2-Propylheptanol, vorzugsweise 2-Ethylhexanol oder 2-Octanol, handelt.

**6.** Verfahren nach einem der vorhergehenden Ansprüche, **dadurch gekennzeichnet, dass** die gesamte Verweilzeit der rohen Reaktionsmischung in Kontakt mit dem katalytisch wirkenden Harz, ausgedrückt durch das Verhältnis von Harzvolumen und Gesamtvolumenströmungsrate der Reaktantenzufuhr, zwischen 2 Stunden und 6 Stunden, vorzugsweise zwischen 2,5 Stunden und 5 Stunden, liegt.

**7.** Verfahren nach einem der vorhergehenden Ansprüche, **dadurch gekennzeichnet, dass** es außerdem eine Reinigungsbehandlung der aus der Rezirkulationsschleife austretenden rohen Reaktionsmischung umfasst, die mindestens folgende Schritte umfasst:

(i) man unterwirft die rohe Reaktionsmischung eine Destillation in einer Topping-Säule zum Erhalt:

∘ eines Stroms, der im Wesentlichen aus den nicht umgesetzten Reaktanten besteht, am Kopf;
∘ einem Strom, der den gewünschten Ester, mit der Säure und dem Alkohol verbundene Verunreinigungen und schwere Nebenprodukte umfasst, am Sumpf;

(ii) man leitet den Sumpfstrom aus der Topping-Säule zu einer Rektifikationssäule zur Trennung:

◦ des gereinigten gewünschten Esters am Kopf;

◦ eines Stroms, der mit der Säure und dem Alkohol verbundene Verunreinigungen und schwere Nebenprodukte umfasst, am Sumpf, wobei dieser Strom an einem Filmverdampfer aufkonzentriert oder in einer Tailing-Säule destilliert wird, um die vorhandenen leichten Verbindungen in die Rektifikationssäule zurückzuführen und den letztendlichen Rückstand von schweren Nebenprodukten zu entfernen.

8. Verfahren nach Anspruch 7, **dadurch gekennzeichnet, dass** die Betriebsbedingungen der Topping-Säule so eingestellt werden, dass mindestens ein Teil der mit der Säure verbundenen Verunreinigungen mitgeschleppt wird.

9. Verfahren nach Anspruch 7 oder 8, **dadurch gekennzeichnet, dass** der Sumpfstrom der Topping-Säule vor der Zuführung zur Rektifikationssäule in Schritt (ii) einem Waschschritt (i)a mit einem wässrigen Strom unterworfen wird.

10. Verfahren nach Anspruch 9, **dadurch gekennzeichnet, dass** der Waschschritt (i)a es ermöglicht, nach Dekantierung

◦ eine wässrige Phase, die die Gesamtheit der mit der (Meth)acrylsäure verbundenen Verunreinigungen umfasst, und

◦ eine organische Phase, die den gewünschten Ester, schwere Nebenprodukte und Spuren von Wasser und (Meth)acrylsäure umfasst;

zu erhalten, wobei die organische Phase nach Entfernung des Wassers dem Schritt (ii) unterworfen wird.

11. Verfahren nach einem der vorhergehenden Ansprüche, **dadurch gekennzeichnet, dass** dem Topping-Schritt (i) ein Schritt der Destillation der rohen Reaktionsmischung in einer Tailing-Säule zur Abtrennung eines Teils der schweren Nebenprodukte vorgeschaltet wird.

**Claims**

1. A method for producing a $C_4$-$C_{10}$ alkyl (meth)acrylate, by direct esterification of (meth)acrylic acid by the corresponding alcohol, the water of reaction being removed in the form of an azeotrope with the esterifying alcohol from a distillation column positioned above the esterification reactor comprising a cationic resin as catalyst, **characterized in that** the alcohol/acid molar ratio at reactor inlet is between 1.4 and 3, and **in that** the crude reaction mixture circulates in a recirculating loop linking together the reactor and the column for removing the water, at a recirculation rate ranging from 6 to 25, expressed by the weight ratio of the stream directed into the loop to the stream sent to a purification treatment.

2. The method as claimed in claim 1, **characterized in that** the alcohol/acid molar ratio at reactor inlet is between 1.8 and 2.3.

3. The method as claimed in claim 1 or 2, **characterized in that** the recirculation rate is between 10 and 20, preferably between 10 and 15, expressed by the weight ratio of the stream directed into the loop to the stream sent to a purification treatment.

4. The method as claimed in any one of the preceding claims, **characterized in that** the acid is acrylic acid.

5. The method as claimed in any one of the preceding claims, **characterized in that** the alcohol is butanol, 2-ethylhexanol, n-octanol, 2-octanol, n-decanol and 2-propylheptanol, preferably 2-ethylhexanol or 2-octanol.

6. The method as claimed in any one of the preceding claims, **characterized in that** the total residence time of the crude reaction mixture in contact with the catalytic resin, expressed by the ratio of the volume of resin to the total volume flow rate of reactant feed, is between 2 hours and 6 hours, preferably between 2.5 hours and 5 hours.

7. The method as claimed in any one of the preceding claims, **characterized in that** it further comprises a purification treatment of the crude reaction mixture leaving the recirculating loop, comprising at least the steps:

(i) submitting the crude reaction mixture to distillation in a topping column to obtain:

◦ at the top, a stream consisting essentially of the unreacted reactants;

○ at the bottom, a stream comprising the required ester, impurities associated with the acid and the alcohol, and heavy byproducts;

(ii) directing the bottom stream from the topping column to a rectification column for separating:

○ at the top, the purified ester that is required;
○ at the bottom, a stream containing impurities associated with the acid and the alcohol and heavy byproducts, which is concentrated on a film evaporator or distilled in a tailing column in order to recycle the light compounds present to the rectification column, and remove the final residue of heavy byproducts.

8. The method as claimed in claim 7, **characterized in that** the operating conditions of the topping column are adjusted to entrain, at least partly, the impurities associated with the acid.

9. The method as claimed in claim 7 or 8, **characterized in that** the bottoms stream from the topping column is submitted to a washing step (i)a with an aqueous stream before it is sent to the rectification column in step (ii).

10. The method as claimed in claim 9, **characterized in that** the washing step (i)a makes it possible to obtain, after decanting,

○ an aqueous phase comprising all of the impurities associated with the (meth)acrylic acid, and
○ an organic phase comprising the required ester, heavy byproducts and traces of water and (meth)acrylic acid;

said organic phase, after removal of the water, being submitted to step (ii).

11. The method as claimed in any one of the preceding claims, **characterized in that** the topping step (i) is preceded by a step of distillation of the crude reaction mixture in a tailing column for separating part of the heavy byproducts.

**Figure 1**

(109)

(108)

(107)

(102)

(101)

(106)

(104)

(105)

(103)

(101 bis)

Figure 2

**Figure 3**

EP 3 174 844 B1

Figure 4

(18)
(6)
(23)
(15)
(21)
(13)
(12)
(22)
(20)
(24)
(33)
(3)
(4)
(32)
(35)
(34)
(36)
(30)
(31)
(1)

RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1219587 A

- US 4748268 A